# EUROPEAN PATENT APPLICATION

(11) **EP 4 272 754 A1**
(43) Date of publication of application: **08.11.2023**
(21) Application number: 22171675.6
(22) Date of filing: 04.05.2022
(51) Int. Cl.: A61K 39/00, C07K 14/725, C12N 15/113

(54) **METHOD FOR PRODUCING CELLS EXPRESSING A CHIMERIC ANTIGEN RECEPTOR (CAR)**

(71) Applicant: Universitätsklinikum Hamburg-Eppendorf, 20246 Hamburg (DE)
(72) Inventor: Ayuk, Ayuketang Francis, 25469 Halstenbek (DE); Fehse, Boris, 22527 Hamburg (DE); Harfmann, Maraike, 24558 Henstedt-Ulzburg (DE); Glow, Dawid, 20251 Hamburg (DE); Sonntag, Tanja, 22851 Norderstedt (DE)
(74) Representative: Uexküll & Stolberg

(57) **Abstract**

The present invention generally relates to the field of cell therapy. In particular, the invention relates to a method for producing a CD45-negative immune effector cell that expresses a chimeric antigen receptor (CAR) directed against CD45. The method comprises (i) transducing the cell with a pre-defined CAR, (ii) inactivating the CD45 gene of the cell, and (iii) selecting the CD45-inactivated and CAR-transduced cell, wherein step (i) can be performed before, simultaneous with or after step (ii). The CD45-negative immune effector cell preferably is a T cell or natural killer (NK) cell. The immune effector cell resulting from the method of the invention can be applied in cell therapy approaches, in particular for the treatment of cancer or immunological diseases. The cell is also particularly useful for stem cell transplantation.

## Description

The present invention generally relates to the field of cell therapy. In particular, the invention relates to a method for producing a CD45-negative immune effector cell that expresses a chimeric antigen receptor (CAR) directed against CD45. The method comprises (i) transducing the cell with a pre-defined CAR, (ii) inactivating the CD45 gene of the cell, and (iii) selecting the CD45-inactivated and CAR-transduced cell in vitro, wherein step (i) can be performed before, simultaneous with or after step (ii). The CD45-negative immune effector cell preferably is a T cell or natural killer (NK) cell. The immune effector cell resulting from the method of the invention can be applied in cell therapy approaches, in particular for the treatment of cancer or immunological diseases. The cell is also particularly useful for stem cell transplantation.

### BACKGROUND OF THE INVENTION

Chimeric antigen receptor (CAR)-based cell therapy represents a new and very effective therapeutic modality, particularly for malignant hematologic diseases [1,2]. Autologous T cells are most commonly used as effector cells, but there are now also successful approaches using allogeneic NK cells, e.g. derived from umbilical cord blood [3]. Previous clinically successful approaches have used CARs against antigens expressed on the target cells, but not on the effector cells [1-3]. When CARs were used against antigens that are also expressed on T cells, fratricide (i.e., mutual destruction of CAR T cells) was observed, as might be expected, and this could be circumvented by knockout of the respective antigens in the effector cells [4-6]. Such knockout of target antigens on effector cells is only possible, if the survival and function of the effector cells are not affected.

CD45 is expressed on almost all healthy and most malignant hematopoietic cells, but not on other, non-hematopoietic cells of the body. This makes CD45 an attractive target for therapeutic purposes. However, the fact that CD45 is expressed on effector cells like T lymphocytes and NK cells makes therapy using anti-CD45-CARs challenging. Given the central role of CD45 in the regulation of effector cell activation, signal transduction, and cytotoxicity [7], a significant loss of function of these cells must be expected upon knockout of CD45. This was confirmed in early work with the RNK-16 cell line (rat), in which wild-type cells, but not CD45-negative mutants were cytotoxically active [8]. In contrast, it was later shown in the mouse model that CD45-negative primary NK cells exhibit defects in activation, signal transduction and cytokine production, whereas cytotoxic activity was preserved in vitro, albeit to a reduced extent [9, 10].

While the mouse data indicate residual functionality of CD45-negative primary NK cells, activation and signal transduction in murine and human NK cells are in part different. For example, murine and human NKG2D differ significantly in structure and the signaling pathways used [11]. Binding and "crosslinking" with anti-CD45 antibodies was found to inhibit the cytotoxic effect of primary human NK cells, but not primary human T cells, although the exact mechanism is yet unclear. The antibodies could stimulate a cytotoxic-responsive function of CD45, or trigger an inhibitory effect [12-15]. Data on functionality of CD45-negative T cells are lacking, mainly because CD45^{-/-} mice develop only very few or no T cells [16]. Indeed, the role and relevance of CD45 does not only differ considerably between species, but also between T and NK cells [17].

A method for preparing cells that are CD45-negative and express a CD45-CAR has been described in international application WO 2017/222593A1. The method comprises four separate steps, namely: (i) CD45 inactivation, (ii) sorting of CD45-negative cells, (iii) expansion of CD45-negative cells, and (iv) transduction of CD45-negative cells with the CD45-CAR. However, due to the number and duration of the individual procedural steps, the method is both time-consuming and labor-intensive. Moreover, knockout of CD45 is only shown in an NK cell line, which is in accordance with already published literature. The challenge of producing CD45-negative CD45-CAR-T cells has not been addressed in the said application.

In view of the foregoing, it was the objective of the invention to develop a method for the efficient production of an immune effector cell that expresses a CAR directed against CD45, which is less time-consuming than the commonly known methods. The method should reliably provide CD45-negative cells that express a CD45-CAR. The present invention addresses this need and provides additional advantages as well. In particular, the invention provides a method which is easy to handle and requires only a few steps for producing an immune effector cell that expresses CD45-CAR. In addition, the method of the present invention is applicable both in T cells and NK cells.

The method of the invention provides CD45-negative CD45CAR-expressing T cells which demonstrate potent functionality in terms of cytotoxicity against CD45-positive target cells, in particular cancer cells. The method is based on the insight that no cell selection or cell sorting step is required after CD45 inactivation and CD45CAR transduction. Instead, if required, the cells are selected exclusively in a final expansion step. During expansion, the CD45-negative cells that express the CD45-CAR eradicate remaining CD45-positive cells within several days by a fratricide mechanism which ultimately results in a substantially homogeneous population of CD45-negative cells that express CD45CAR.

### DESCRIPTION OF THE INVENTION

Thus, in a first aspect, the present invention provides a method of producing a CD45-negative immune effector cell that expresses a chimeric antigen receptor (CAR) directed against CD45, said method comprising
(a) providing a CD45-positive immune effector cell,
(b) modifying the immune effector cell such that it expresses a CAR directed against CD45,
(c) inactivating the endogenous CD45 gene of the immune effector cell, and
(d) incubating to select a CD45-negative immune effector cell that expresses the CAR directed against CD45 in vitro,
wherein step (b) can be performed either before, simultaneous with or after step (c), and wherein no selection or sorting step is performed after step (a) and prior to step (d).

The method of the invention is directed to the manufacturing of genetically modified immune effector cells which are useful for therapeutic purposes. In a first step of the method, i.e. step (a) of the method of the invention, a CD45-positive immune effector cell is provided. As used herein, an "immune effector cell" refers to a cell that is capable of modulating or effecting an immune response in a mammal, preferably a human. Suitable immune effector cells comprise, but are not limited to dendritic cells, monocytes, macrophages and lymphocytes. Suitable lymphocytes may comprise B cells, NK cells and T cells. These cells can be either directly isolated from blood or derived from suitable progenitor cells, such as cord blood, hematopoietic stem cells, or induced pluripotent stem cells. In a preferred embodiment, the CD45-positive immune effector cell provided in step (a) of the method of the invention is an NK cell or T cell. In a particularly preferred embodiment, the CD45-positive immune effector cell provided in step (a) of the method of the invention is a T cell. The cell may be autologous in respect of the subject that receives the modified cell, i.e. the cell provided in step (a) of the method of the invention has previously been obtained from said subject. Alternatively, the cell may be allogeneic in respect of the subject that receives the modified cell, i.e. the cell provided in step (a) of the method of the invention has previously been obtained from a genetically non-identical subject that belongs to the same species as the subject that receives the modified cell. It is particularly preferred that the CD45-positive immune effector cell to be modified by the method of the invention is an autologous cell.

For example, autologous primary T cells may be conveniently obtained from a subject by collecting a whole-blood sample or by leukapheresis. From this sample, the buffy coat (BC) is isolated according to methods commonly known in the art and described below in the example part. In a subsequent step, peripheral blood mononuclear cells (PBMCs) are isolated from the BC and may be activated in vitro according to commonly known methods, e.g. by the addition of a suitable agent such as T Cell TransAct^{™} (Miltenyi Biotec, Bergisch Gladbach, Germany) and appropriate cytokines, e.g. human interleukin (IL)-2 or IL-7 + IL-15. Alternatively, autologous primary NK cells may be obtained from PBMCs that are isolated from a BC by using an appropriate isolation kit e.g. Dynabeads^{™} Untouched^{™} Human NK cells (Thermo Fisher Scientific, Schwerte, Germany). Activation of the cells can be performed with commonly known methods, e.g. by the addition of human IL-2.

In step (b) of the above method, the immune effector cell is modified such that it expresses a CAR directed against CD45. The modification can be achieved in several ways. For example, the genome of the immune effector cell can be modified by the inclusion of a nucleotide sequence that codes for a CD45CAR construct using genome-editing techniques. Alternatively, a nucleotide sequence encoding a CD45CAR construct can be introduced into immune effector cell by transfection with a non-viral vector (e.g. a transposon) or by transduction with a viral vector, preferably a retroviral vector (e.g. a γ-retroviral, α-retroviral, or lentiviral vector). A suitable construct for use in the method of the invention is disclosed, for example, in WO2017/222593A1 and comprises an anti-CD45 single-chain variable fragment (scFv), CD8-derived hinge (H) and transmembrane (TM) regions, and tandem CD28 and 4-1BB co-activation domains linked to the CD3 signaling domain. The construct uses a strong spleen focus forming virus promoter (SFFV) and a CD8 leader sequence. Transduction is achieved in WO2017/222593A1 by a lentiviral vector system.

In step (c) of the above method, the endogenous CD45 gene of the immune effector cell is inactivated. Gene inactivation can be achieved in a number of suitable ways, all of which are well known in the art. The gene of interest may be disrupted by the inclusion of an unrelated nucleotide sequence, which causes a shift in the reading frame. Alternatively, part of the nucleotide sequence of the gene may be deleted. Gene inactivation can be achieved, e.g. by homologous recombination or gene editing using suitable designer enzymes, such as CRISPR/CAS, TALENs or others. In a preferred embodiment, the inactivation step (c) is performed by CRISPR/CAS gene editing.

In one embodiment, the CAR modification step (b), and preferably the transduction of the cell, is performed before CD45 inactivation step (c). For example, the CAR modification step (b) can be carried out 12-36 hours before performing inactivation step (c). Preferably, the time period between modification step (b) and inactivation step (c) is at least 12 hours, at least 14 hours, at least 16 hours, at least 18 hours, at least 20 hours, at least 22 hours, at least 24 hours, at least 26 hours, at least 28 hours, at least 30hours, at least 32 hours, or at least 34 hours.

It is also important to note that in humans there are at least eight different isoforms of CD45 at least six of which are expressed by different blood cells at reasonable levels [7]. In one embodiment, the CD45CAR targets the RABC isoform of human CD45 (SEQ ID NO:1). In another embodiment, the CD45CAR targets the R0 isoform of human CD45 (SEQ ID NO:2). In yet another embodiment, the CD45CAR targets the RAB isoform of human CD45 (SEQ ID NO:3). In yet another embodiment, the CD45CAR targets the RAC isoform of human CD45 (SEQ ID NO:4). In yet another embodiment, the CD45CAR targets the RBC isoform of human CD45 (SEQ ID NO:5). In yet another embodiment, the CD45CAR targets the RA isoform of human CD45 (SEQ ID NO:6). In yet another embodiment, the CD45CAR targets the RB isoform of human CD45 (SEQ ID NO:7). In yet another embodiment, the CD45CAR targets the RC isoform of human CD45 (SEQ ID NO:8).

In step (d) of the above method, the CD45-negative immune effector cell that expresses the CAR directed against CD45 (CD45-negative CD45CAR cells) is selected which means that remaining CD45-positive cells which are present in the population are eradicated. The selection step can be performed by expanding the cells in vitro. For this, the cells are regularly (e.g. 3 times weekly) provided with fresh medium (e.g. medium that is composed as described in the example part). If necessary, the cells are moved to other cell culture vessels of the appropriate size. During the in vitro expansion step, any cells obtained from the manufacturing method, which are still CD45-positive, i.e. cells which have escaped CD45 inactivation, will be killed by the CD45CAR cells. The in vitro expansion step can be performed for a period between 20 minutes and 20 days. Preferably, the in vitro expansion step is performed for at least 30 minutes, at least 60 minutes, at least 120 minutes, at least 240 minutes, at least 300 minutes, or at least 360 minutes. More preferably, the in vitro expansion step is performed for 2 days, at least 4 days, at least 6 days, at least 8 days, at least 10 days, at least 12 days, at least 14 days, at least 16 days, at least 18 days, or at least 20 days. In another preferred embodiment, the in vitro expansion step is performed for a time period that is sufficient to reduce the proportion of CD45-positive cells in the population to less than 50%, and preferably less than 40%, less than 30%, less than 20%, or less than 10% of the cell population obtained from step (d), as determined by flow cytometry. More preferably, the proportion of CD45-positive cells in the population is reduced to less than 8%, less than 6%, less than 4%, less than 2% or less than 1% of the overall number of cells present cell population obtained from step (d), as determined by flow cytometry. After the in vitro expansion step, the cells can be either administered to a subject in need of the cells or stored until further use, e.g. by freezing.

Alternatively, the selection step can be performed by administering the cells obtained after performing steps (a)-(c) of the above method into a subject. In this case, the selection is that eradicates CD45-positive cells is achieved within the patient's body.

In a particular embodiment, the method of the invention comprises a further step (e), in which the immune effector cell obtained from step (d) is cryopreserved, e.g. by freezing the cells. For example, the cells obtained from step (d) can be frozen to a temperature -80°C and stored until further use, e.g. by administration into the patient.

In a particular embodiment, the method of the invention comprises a further step (f), in which the immune effector cell obtained from step (d) or (e) is tested for CD45 and CD45-CAR expression, preferably at the end of the expansion, e.g. after 2 days, after 4 days, after 6 days, after 8 days, after 10 days, after 12 days, after 14 days, after 16 days, after 18 days, or after 20 days. CD45 and CD45-CAR expression can be detected for example by using antibodies directed against CD45 and CD45-CAR, respectively. Preferably, CD45 and CD45-CAR expression is analyzed by flow cytometry.

In one embodiment, the immune effector cell expresses more than one CAR. For example, the immune effector cell may express a CD45-CAR and a second CAR which is directed to a protein selected from the group consisting of B Cell Maturation Antigen (BCMA), Kappa or lambda light chain, Lewis Y, CLL-1, HLA-A1, HLA-A2, CD5, CD7, CD10, CD11a, CD19, CD20, CD22, CD23, CD26, CD30, CD33, CD34, CD37, CD38, CD43, CD44v6, CD56, CD59, CD79a, CD79b, CD90, CD117, CD16, CD123 and CD133. IN a preferred embodiment, the immune effector cell may express a CD45-CAR and a CD19-CAR. Accordingly, the immune effector cell has been modified such that it expresses a CAR directed against CD45 and a CAR directed against CD19. In such cases, the one or more second CAR may be an activating CAR, which means that it triggers an activating signal of the effector cell (e.g. a proliferation or cytotoxicity signal), or it may be an inhibitory CAR, which means that it triggers an inhibitory signal of the effector cell that leads to reduction or inhibition of proliferation or cytotoxicity. This can be achieved, e.g., by transducing the immune effector cell, such as a T cell or an NK cell, with two separate viral vectors that code for CD45-CAR and, for example, CD19-CAR, respectively.

In another embodiment, the cells produced by the method of the invention are additionally modified to allow their selective elimination in case of unwanted side effects. These modifications might be based on suitable genetic elements, e.g. prodrug-converting enzymes or suitable receptor molecules, facilitating cell suicide (apoptosis) after application of corresponding prodrugs or ligands. Alternatively, these modifications might be receptor/cell-surface molecules as potential targets of antibody-dependent cellular cytotoxicity.

In another embodiment, instead of the CD45CAR, the immune cells express a universal CAR, whose activity depends on the presence of a soluble adapter, which links the CAR to CD45 on the target cell. The generation of a universal CAR (UniCAR) is described, for example, in Meyer et al. 2021.

### BRIEF DESCRIPTION OF THE FIGURES

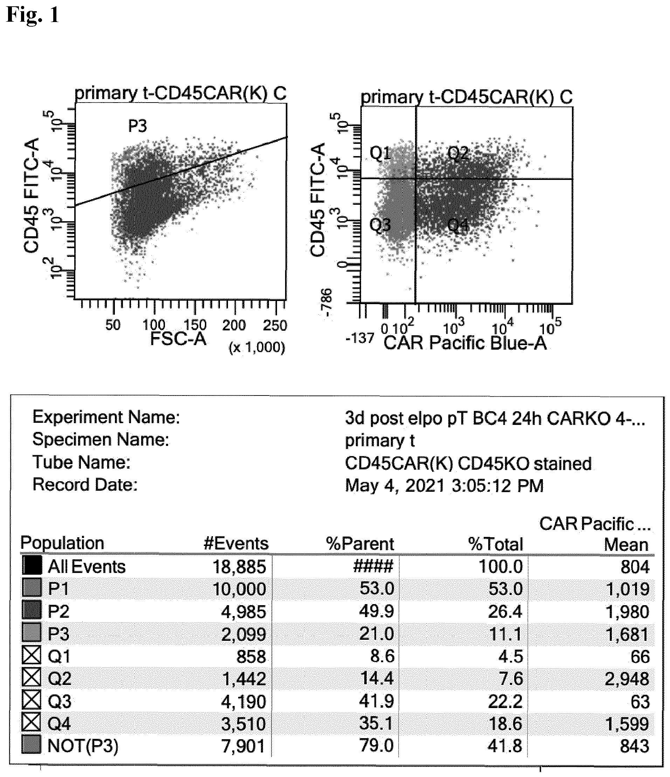

**Figure 1** shows the results from the flow cytometry analysis 3 days after CD45KO. Primary T cells were transduced with CD45CAR, and CD45KO was performed 24h later. CD45 was stained with FITC-labelled antibody, and CD45CAR detectable in the Pacific Blue channel via co-expressed blue-fluorescence protein (BFP). Q2+Q4 represent CAR-positive T cells, making up about 50% of the cell population. Q4 represents CD45KO/CD45CAR-T cells (35.1%). 3 days after CD45KO only 8.6% (Q1) of the cells are still CD45-positive and CD45CAR-negative.

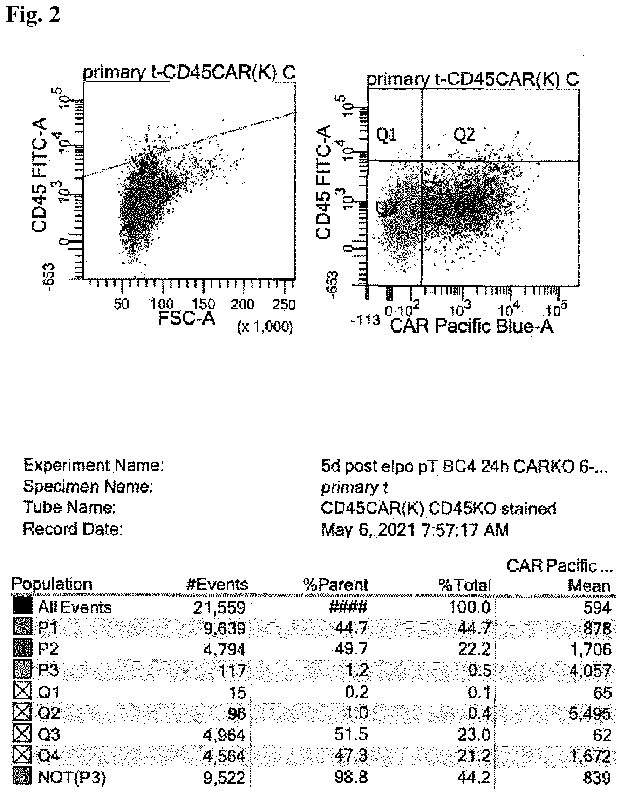

**Figure 2** shows the results from the flow cytometry analysis 5 days after CD45KO. Primary T cells were transduced with CD45CAR, and CD45KO was performed 24h later. CD45 was stained with FITC-labelled antibody, and CD45CAR detectable in the Pacific Blue channel via co-expressed blue-fluorescence protein (BFP). Q4 represents CD45KO/CD45CAR-T cells (47.3%). 5 days after CD45KO only 0.2% (Q1) of the cells are still CD45-positive and CD45CAR-negative.

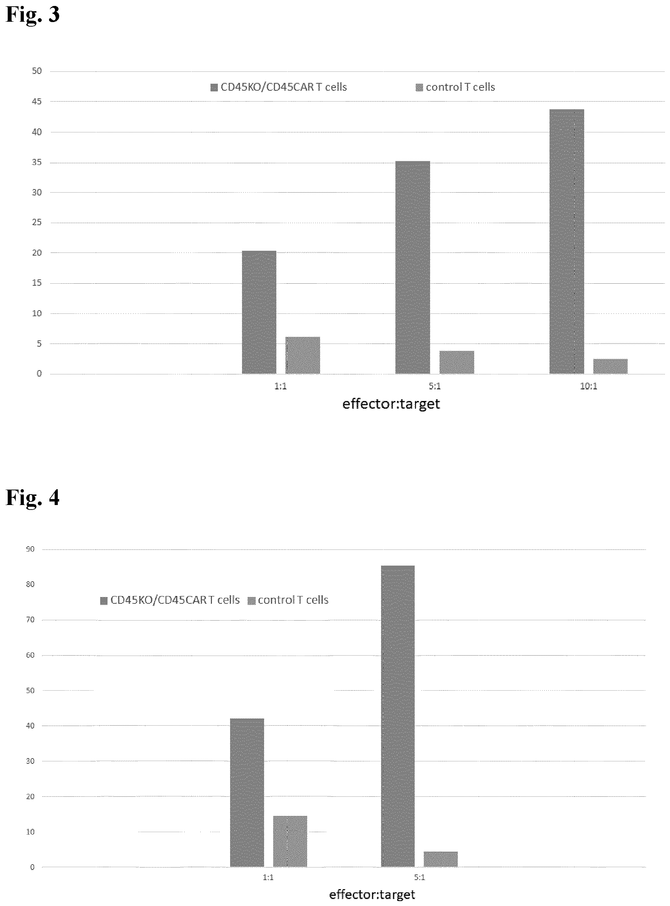

**Figure 3** shows the results of the luciferase killing assay after 4 hours of incubation. The Y-axis indicates killing rates [%] of Ramos-Luc2 cells (human lymphoma cell line) with CD45 primary CAR-T-cells (Buffy coat No. 4: day 10 of production = day 5 after CD45KO) at different effector-to-target ratios (X-axis).
**Figure 4** shows the results of the luciferase assay after 4 hours of incubation. The Y-axis indicates killing rates [%] of Ramos-Luc2 cells with CD45 primary CAR-T-cells (Buffy coat No. 5: day 22 of production = day 17 after KO) at different effector-to-target ratios (X-axis).

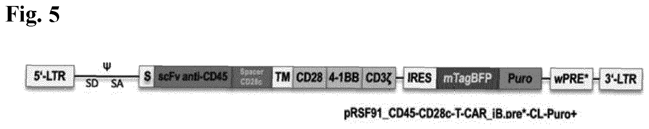

**Figure 5** shows a schematic presentation (not to scale) of the tested γ-retroviral CAR vectors. (LTR = long terminal repeat; SD = splice donor; SA = splice acceptor, S: leader; scFv = single chain variable fragment which is codon-optimized; H23c: IgG4-hingeCH2CH3.cystein = long spacer; CD28c: CD28.cystein = short spacer; TM = transmembrane; CD28 & 4-1BB & CD3zeta = intracellular signal transduction for T cell activation; IRES = internal ribosomal entry site; mTagBFP = monomeric blue fluorescent protein; Puro = puromycin resistance; wPRE = WHP (woodchuck hepatitis virus) posttranscriptional response element.

### EXAMPLES

The present invention is further described in more detail by the following examples which are only provided for illustrating the invention and which are not to be construed as limiting the scope of the invention. The following material and methods were used in the Examples.

### Example 1: Isolation and activation of primary T cells

Peripheral blood mononuclear cells (PBMCs) were isolated from buffy coats (BC) via density gradient using Secoll^{™} Lymphocyte Separation Media (Serana, Pessin, Germany). The buffy coats are leftovers after whole-blood donations of healthy donors and were kindly provided by the Institute of Transfusion Medicine, University Medical Center Hamburg- Eppendorf, Hamburg, Germany.

First, the BC (50-65 ml) was diluted 1:2 with sterile PBS buffer. 15 mL Secoll^{™} were placed in 50 ml Falcon^{™} tubes and carefully overlaid with the BC/PBS mixture (15-35 ml). After centrifugation for 30 min (1000 x g, room temperature [RT], brake turned off), the interphase layer containing the PBMCs was harvested. The cells were washed three times with sterile PBS, each time with a centrifugation step (400 x g, 7 min, RT). Finally, the PBMC pellet was resuspended in 5 ml TexMACS^{™} medium (Miltenyi Biotec).

To obtain primary T cells for further experiments and cultivation, the freshly isolated PBMCs were activated with CD3/CD28 agonist-conjugated beads, T Cell TransAct^{™} for human T cells (Miltenyi Biotec). The PBMCs were seeded in a 6-well suspension cell plate at a cell concentration of 1.5 x 10⁶ /ml. T cell TransAct^{™} was added according to the manufacturer's instructions at a 1:100 ratio. Human recombinant IL-2 was added to the medium at 200 IU/ml. The primary T cells were cultured in TexMACS Medium^{™} supplemented with 3% human serum (Sigma-Aldrich, Steinheim, Germany) and 200 U/ml hIL-2. Cell density was maintained at approximately 1-2 x 10⁶ per ml, and cells were split if necessary. Cell cultures were provided fresh medium according to their needs and medium consumption. Cell incubation was under standard conditions of 37°C, 100% relative humidity, 5% CO₂.

### Example 2: Production of retroviral particles

Retroviral particles harboring the CD45CAR vector (pRSF91_CD45-CD28c-T-CAR _iB_prePuroR, SEQ ID NO:9) were produced by transiently transfecting HEK293T cells using the calcium phosphate method established in our lab [18]. This vector is based on the SF91 γ-retroviral vector backbone [19] and encodes a standard CAR construct [20] as shown in Figure 5. The CD45-directed scFv in the CAR [based on 21] is encoded by a novel, codon-optimized DNA sequence designed by the applicants.

The following plasmids were used: 6 µg phCMV.MLV.gp, 4 µg phCMV-GALV-C4070A and 10 µg vector plasmid pRSF91_CD45-CD28c-T-CAR _iB_prePuroR [18-20]. In the afternoon before transfection 5 x 10⁶ HEK293T cells were seeded in 10 ml medium (DMEM high glucose (4.5 g/1) GlutaMAX^{™} Supplement pyruvate supplemented with penicillin-streptomycin (100 U/ml and 100 µg/ml), 10% heat inactivated FCS, 25 mM HEPES) in a 10-cm cell culture dish for adherent cells. In the morning the plasmids were thawed to room temperature. H₂O and plasmids were mixed in a 1.5-ml Eppendorf tube to a total volume of 450 µl. 500 µl 2X HBS buffer were pipetted into 15-ml Falcon tubes. Finally, 50 µl of 2.5M calcium chloride (CaCl2) were added to the premixed plasmids and the whole mixture vortexed. Directly afterwards the mixture was added dropwise to the 2X HBS buffer while constantly blowing air into the buffer with a 2 ml serological pipette on maximum blow-out power. The medium of the HEK293T cells was exchanged to 10 ml 293T medium containing 25 µM chloroquine. After incubation for 20 min at RT the plasmid-buffer mix was equally and dropwise spread over the 293T cell plate. Last, the plate was carefully moved in an "eight-shape" to further distribute the plasmids and incubated at 37°C. After at least 6 h the medium was changed to 8 ml of the standard 293T medium without chloroquine. The supernatant containing the viral particles was harvested every 12 h for 3 times in total. The vector-containing supernatant was harvested using a syringe and filtered through a 0.45-µm syringe-filter. In between the different harvest time points the vector supernatant was kept at 4°C. After the last harvest (36 h) the pooled supernatant was concentrated via centrifugation (8000 xg, 4°C) overnight or at least for 6 h. The medium above the particle pellet was discarded leaving 300-400 µl volume. Finally, the pellet was resuspended and stored in aliquots at -80°C.

### Example 3: Transduction of primary T cells and subsequent CD45 gene knockout

Human primary T cells obtained from Example 1 were transduced with GALV-pseudotyped, γ-retroviral particles [18] to insert the gene encoding for the chimeric antigen receptor (CAR) (scFv anti-CD45, SEQ ID NO:10) in conjunction with the BFP marker gene (for easy detection of transduced cells). The cells were activated with T Cell TransAct^{™} for three days as described above.

Before transduction on day 4 after activation, suspension plates (6-well) were coated with Retronectin^{™} (Takara Bio, Europe AB, Göteborg Sweden). The plates were prepared on the day before transduction by adding 2 mL Retronectin^{™} per well and incubation at 4°C overnight, or on the same day with incubation at RT for 2 h. Afterward, the Retronectin^{™} was removed, 2 ml blocking buffer (PBS + 2% BSA) were added per well, and the plate was incubated for 30 minutes at RT. Next, the wells were washed twice with HBSS (1x) + 2.5% HEPES. The supernatant containing the viral-vector particles harboring the CD45CAR vector (pRSF91_CD45-CD28c-T-CAR _iB_prePuroR) was added in TexMACS^{™} in a total volume of 1 mL to provide a multiplicity of infection (MOI) of 10. The plate was centrifuged for 1 h (1000 x g, 4°C) to pre-load vector particles to the Retronectin^{™}. The medium containing non-bound vector particles was removed and 1 x 10⁶ human primary T cells were added in 2 ml T cell medium. The transduced T cells were harvested by carefully removing from the plate 24 hours after transduction and counted.

Subsequently, the transduced cells were subjected to the CD45 knockout. To build CRISPR-Cas9-sgRNA RNP-complexes, the Cas9 protein TrueCut^{™} v2 (Thermo Fisher Scientific, Schwerte, Germany) was mixed in a PCR tube with the sgRNA targeting the CD45 gene. The sgRNA was directed to nucleotides 261-279 (protospacer/crRNA) with the Protospacer adjacent motif (PAM) at nucleotides 280-282 of the CD45 gene set forth in SEQ ID NO:11. The sequence of nucleotides 261-282 of the CD45 gene are separately provided as SEQ ID NO:12. The molar ratio was 1:2.5 of Cas9 to sgRNA in a total volume of approximately 2 µl. The mixture was incubated for 10 min at RT.

The T cells were prepared in a concentration of 400,000 cells/15 µl OPTI-MEM^{®} (Thermo Fisher Scientific) after a washing step (centrifugation 310 x g, 5 min, RT). 15 µl cells were added to the pre-built RNP-complexes and directly transferred to a 1-mm cuvette without causing any air bubbles. The cell-RNP mixture was electroporated with the GenePulser Xcell^{™} (BioRad, Munich, Germany) under the following conditions: 90 V, square-wave pulses, 10 ms pulse length, 3 pulses with 0.1 ms pause in-between. The cells were washed out of the cuvette using pre-warmed T cell medium and cultured in 1 ml in a 24-well suspension plate (37 °C). Three days after electroporation, the cells were analyzed by flow cytometry. The results of this analysis are shown in Figure 1. Cells were maintained in culture medium and used for cytotoxicity assays on day 10 (Figure 3)

### Example 4: CD45 gene knockout in primary T cells and subsequent transduction

Human primary T cells obtained from Example 1 were used for a CD45 knockout. To build CRISPRCas9-sgRNA RNP-complexes, the Cas9 protein TrueCut^{™} v2 (Thermo Fisher Scientific, Schwerte, Germany) was mixed in a PCR tube with the sgRNA targeting the CD45 gene. The molar ratio was 1:2.5 of Cas9 to sgRNA in a total volume of approximately 2 µl. The mixture was incubated for 10 min at RT.

The human primary T cells were prepared in a concentration of 400,000 cells/15 µl OPTI-MEM^{®} (Thermo Fisher Scientific) after a washing step (centrifugation 310 x g, 5 min, RT). 15 µl cells were added to the pre-built RNP-complexes and directly transferred to a 1-mm cuvette without causing any air bubbles. The cell-RNP mixture was electroporated with the GenePulser Xcell^{™} (BioRad, Munich, Germany) under the following conditions: 90 V, square-wave pulses, 10 ms pulse length, 3 pulses with 0.1 ms pause in-between. The cells were washed out of the cuvette using pre-warmed T cell medium and cultured in 1 ml in a 24-well suspension plate (37 °C).

Subsequently, the cells obtained from the CD45 knockout procedure were transduced with retroviral-vector particles to insert the gene encoding for the chimeric antigen receptor (CAR) in conjunction with the BFP marker gene (for easy detection of transduced cells). The cells were activated with T Cell TransAct^{™} for three days as described above.

Before transduction, (6-well) suspension plates were coated with Retronectin^{™} (Takara Bio, Europe AB, Göteborg Sweden). The plates were prepared on the day before transduction by adding 2 mL Retronectin^{™} per well and incubation at 4°C overnight, or on the same day with incubation at RT for 2 h. Afterward, the Retronectin^{™} was removed, 2 ml blocking buffer (PBS + 2% BSA) were added per well, and the plate was incubated for 30 minutes at RT. Next, the wells were washed twice with HBSS (1x) + 2.5% HEPES. The supernatant containing the viral-vector particles harboring the CD45CAR vector (pRSF91_CD45-CD28c-T-CAR _iB_prePuroR) was added in TexMACS^{™} in a total volume of 1 mL to provide a multiplicity of infection (MOI) of 10. The plate was centrifuged for 1 h (1000 x g, 4°C) to pre-load vector particles onto the Retronectin^{™}. The medium containing non-bound vector particles was removed and 1 x 10⁶ human primary T cells were added in 2 ml T cell medium. 24 hours after transduction, by carefully removing them from the plate the transduced T cells were harvested and counted. Cells were maintained in culture medium and used for cytotoxicity assays on day 22 (Figure 4)

### Example 5: Luciferase-based killing assay

The cells obtained from Examples 3 and 4 were used in a luciferase-based killing assay to test the killing ability of the CD45KO-CD45CAR-T cells. Ramos cells with luciferase activity were exploited as target cells after confirming their CD45 expression via antibody staining and flow cytometry.

The Ramos cell line was cultured in RPMI Medium 1640 (Gibco / Thermo Fisher Scientific, Schwerte Germany) supplemented with penicillin-streptomycin (100 U/ml and 100 µg/ml), L-glutamine (2 mM), 25 mM HEPES (1M), 13% heat-inactivated FCS, and 1 µg/ml puromycin. Cell cultures were provided fresh medium according to their needs and medium consumption. They were regularly split to ensure optimal density. Cell incubation was under standard conditions: 37°C, 100% relative humidity, 5% CO2.

On the day of the killing assay the CD45KOCD45CAR-T cells were analyzed by flow cytometry to check for the rate of CAR+ cells based on BFP-expression. This rate was later used for calculation of the target:effector ratios to assess Ramos:CD45CAR-T ratios.

The cells were also analyzed for CD45 expression by staining with a monoclonal CD45 Antibody (anti-human, FITC, REAfinity^{™}, Miltenyi Biotec) according to the manufacturer protocol. Flow cytometry was performed on the FACSCanto^{™} II Cell Analyzer (BD Biosciences, Heidelberg, Germany).

The viability of the Ramos cells could be detected after addition of luciferin by measuring the bioluminescence with a plate reader. Target and effector cells were counted and seeded together in a white 96-well-OptiPlate^{™} (Perkin Elmer, Waltham, USA). 10,000 target cells were seeded in 100 µl/ well in effector cell medium, and effector cells were added at different effector:target (E-T) ratios. Effector cell medium was added to provide a total volume of 200 µL in each well. As zero control, "target cells only" were seeded in 200 µl effector cell medium to obtain the baseline luminescence value. As non-specific activity control, Ramos cells were co-cultured with untransduced T cells in the same E-T ratios as for the CAR-transduced effector T cells.

The plate was incubated at 37°C in the cell incubator for 4 h. After this, the plate was centrifuged at 1000 x g for 5 min at RT to pellet the cells. 100 µl supernatant were carefully removed with a multi-channel pipette. D-Luciferin Firefly (Biosynth, Staad, Switzerland) was diluted in PBS (10 ng in 100 µl PBS). Then, the cell pellets were resuspended in 100 µl of the D-Luciferin dilution per well and incubated for exactly 20 min in the dark. Luminescence was analyzed with the plate reader Infinite^{®}200 pro (Tecan, Männedorf, Switzerland) using 2000 ms integration time.

Results: The results from the killing assay for cells obtained from Example 3 (transduction followed by CD45 knockout) are shown in Figure 3. The results from the killing assay for cells obtained from Example 4 (CD45 knockout followed by transduction) are shown in Figure 4. Figures 3 and 4 depict the cytotoxicity of CD45KO/CD45CAR-T cells against CD45 expressing Ramos cells as measured at day 10 (Figure 3) and at day 22 (Figure 4) of production compared to untransduced control T cells. As evident, already after four hours of co-culture, potent cytotoxicity was observed for CD45KO/CD45CAR-T cells, even at an effector-target (E-T) ratio of 1:1; specific killing reached >40% at an E-T ratio 10:1 (Figure 3). The even stronger cytotoxicity of CD45KO/CD45CAR-T cells observed on day 22 of production (See Figure 4) indicates that cytotoxic potential is well preserved even after 2-3 weeks of in vitro culturing and expansion. Increased activity is most likely due to the eradication of any remaining CD45-expressing T cells that were not only targets of fratricide (thus binding some of the activity of the CD45CAR-T cells), but also less active as CD45-CAR expressing effector cells.

### LITERATURE

1. Schuster SJ, Bishop MR, Tam CS et al., Tisagenlecleucel in Adult Relapsed or Refractory Diffuse Large B-Cell Lymphoma. N Engl J Med. 2019; 380:45-56.
2. Neelapu SS, Locke FL, Bartlett NL, et al., Axicabtagene Ciloleucel CAR T-Cell Therapy in Refractory Large B-Cell Lymphoma. N Engl J Med. 2017;377:2531-2544.
3. Liu E, Marin D, Banerjee P, et al., Use of CAR-Transduced Natural Killer Cells in CD19-Positive Lymphoid Tumors. N Engl J Med. 2020;382:545-553.
4. Raikar SS. Fleischer LC, Moot R, et al., Development of chimeric antigen receptors targeting T-cell malignancies using two structurally different anti-CD5 antigen binding domains in NK and CRISPR-edited T cell lines. Oncoimmunology. 2017;7:e1407898.
5. Gomes-Silva D, Srinivasan M. Sharma S et al., CD7-edited T cells expressing a CD7-specific CAR for the therapy of T-cell malignancies. Blood. 2017;130:285-296.
6. Rasaiyaah J, Georgiadis C. Preece R, Mock U. Qasim W. TCRaβ/CD3 disruption enables CD3-specific antileukemic T cell immunotherapy. JCI Insight. 2018 Jul 12;3(13):e99442.
7. Holmes N. CD45: all is not yet crystal clear. Immunology. 2006;117:145-55.
8. Bell GM. Dethloff GM, lmboden JB. CD45-negative mutants of a rat natural killer cell line fail to lyse tumor target cells. J Immunol. 1993;151:3646-53.
9. Hesslein DG, Takaki R, Hermiston ML, Weiss A, Lanier LL Dysregulation of signaling pathways in CD45-deficient NK cells leads to differentially regulated cytotoxicity and cytokine production. Proc Natl Acad Sci USA. 2006;103:7012-7.
10. Huntington ND, Xu Y, Nutt SL, Tarlinton DM. A requirement for CD45 distinguishes Ly49D-mediated cytokine and chemokine production from killing in primary natural killer cells J Exp Med. 2005;201:1421-33.
11. Rosen DB, Araki M, Hamerman JA, Chen T. Yamamura T, Lanier LL A Structural basis for the association of DAP12 with mouse, but not human. NKG2D. J Immunol. 2004; 173:2470-8.
12. Sparrow RL, McKenzie IF. A function for human T200 in natural killer cytolysis. Transplantation. 1983;36:166-71.
13. Starling GC, Davidson SE, McKenzie JL, Hart DN. Inhibition of natural killer-cell mediated cytolysis with monoclonal antibodies to restricted and non-restricted epitopes of the leucocyte common antigen. Immunology. 1987;61:351-6.
14. Starling GC. Hart DN. CD45 molecule cross-linking inhibits natural killer cell-mediated lysis independently of lytic triggering. Immunology. 1990;71:190-5.
15. Poggi A, Pardi R. Pella N et al., CD45-mediated regulation of LFA1 function in human natural killer cells. Anti-CD45 monoclonal antibodies inhibit the calcium mobilization induced via LFA1 molecules. Eur J Immunol. 1993;23:2454-63.
16. Ogilvy S, Louis-Dit-Sully C. Cooper J, Cassady RL, Alexander DR. Holmes N. Either of the CD45R and CD45RO isoforms are effective in restoring T cell. but not B cell. development and function in CD45-null mice. J Immunol. 2003;171:1792-800.
17. Martin SM, Mehta IK, Yokoyama WM, Thomas ML, Lorenz RG. Development of intestinal intraepithelial lymphocytes, NK cells, and NK 1.1+ T cells in CD45-deficient mice. J Immunol. 2001;166:6066-73.
18. Mirow M, Schwarze LI, Fehse B, Riecken K. Efficient Pseudotyping of Different Retroviral Vectors Using a Novel, Codon-Optimized Gene for Chimeric GALV Envelope. Viruses. 2021;13:1471.
19. Hildinger M, Abel KL, OstertagW, Baum C. Design of 5' untranslated sequences in retroviral vectors developed for medical use. J Virol. 1999;73:4083-4089.
20. Hambach J, Riecken K, Cichutek S, Schütze K, Albrecht B, Petry K, Röckendorf JL Baum N, Kröger, N Hansen T, Schuch G, Haag F, Adam G, Fehse B, Bannas P, Koch-Nolte F. Targeting CD38-Expressing Multiple Myeloma and Burkitt Lymphoma Cells In Vitro with Nanobody-Based Chimeric Antigen Receptors (Nb-CARs). Cells 2020; 9: 321.
21. Lin Y, Pagel JM, Axworthy D, Pantelias A, Hedin N, Press OW. A genetically engineered anti-CD45 single-chain antibody-streptavidin fusion protein for pretargeted radioimmunotherapy of hematologic malignancies. Cancer Res. 2006; 66:3884-92.

## Claims

1. Method of producing a CD45-negative immune effector cell that expresses a chimeric antigen receptor (CAR) directed against CD45, said method comprising
(a) providing a CD45-positive immune effector cell,
(b) modifying the immune effector cell such that it expresses a CAR directed against CD45,
(c) inactivating the endogenous CD45 gene of the immune effector cell, and
(d) incubating to select a CD45-negative immune effector cell that expresses the CAR directed against CD45,
wherein step (b) can be performed either before, simultaneous with or after step (c) and wherein no selection or sorting step is performed after step (a) and prior to step (d).

2. Method of claim 1, wherein the immune effector cell is a T cell.

3. Method of claim 1, wherein the immune effector cell is a natural killer (NK) cell.

4. Method of claim 2 or 3, wherein said T cell or NK cell is an autologous or allogeneic cell.

5. Method of any of claims 1-4, wherein modification step (b) is performed before inactivation step (c).

6. Method of claim 5, wherein the modification step (b) is performed 12-36 hours before inactivation step (c).

7. Method of any of claims 1-6, wherein inactivation step (c) is performed by targeted genome editing, and preferably by using CRISPR/CAS or TALENs.

8. Method of any of claims 1-7, wherein modification step (b) is performed with a viral or non-viral vector.

9. Method of any of claims 1-8, wherein step (d) is performed for 6 hours-20 days.

10. Method of any of claims 1-9, wherein the method further comprises step (e) in which the immune effector cell obtained from step (d) is tested for CD45 and CD45-CAR expression.

11. Method of any of claims 1-10, wherein less than 10% of the cell population obtained from step (d) express CD45.

12. Method of any of claims 1-11, wherein at least 30% of the cell population obtained from step (d) express CD45-CAR

13. Method of any of claims 1-12, wherein the immune effector cell expresses a first CAR that targets CD45 and a second CAR that target a protein other than CD45.

14. Method of claim 13, wherein the second CAR targets CD19.

15. Method of claim 13 or 14, wherein the second CAR provides an activating or an inhibitory signal.
